# FASCICULE DE BREVET EUROPEEN

(11) **EP 0 347 313 B1**
(45) Date de publication et mention de la délivrance du brevet: **15.12.1993**
(21) Numéro de dépôt: 89401661.7
(22) Date de dépôt: 14.06.1989
(51) Int. Cl.: C07C 217/54

(54) **Ethers de la 2-amino-7-hydroxytétraline**
2-Amino-7-hydroxytetralin-ether
2-Amino-7-hydroxytetraline ethers

(30) Priorité: 14.06.1988 FR 8807948
(43) Date de publication de la demande: 20.12.1989
(73) Titulaire: ELF SANOFI, 75008 Paris (FR); MIDY S.p.A., 20137 Milano (IT)
(72) Inventeur: Boigegrain, Robert, F-34170 Castelnau le Lez (FR); Cecchi, Roberto, I-20075 Lodi Milano (IT); Boveri, Sergio, I-15057 Tortona (Alessandria) (IT)
(74) Mandataire: Gillard, Marie-Louise

(56) Documents cités:
- EP-A- 0 211 721
- EP-A- 0 253 257

## Description

La présente invention concerne des éthers de la 2-amino-7-hydroxytétraline, un procédé et des intermédiaires pour leur préparation et leur utilisation pour la synthèse de phényléthanolaminotétralines pharmacologiquement actives.

La demande EP 211721 décrit des phényléthanolaminotétralines de formule 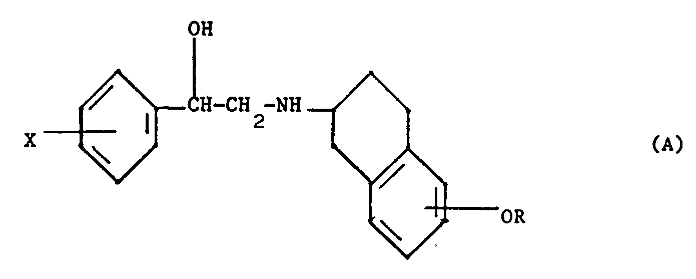
dans laquelle X représente l'hydrogène, un halogène, un groupe trifluorométhyle ou un groupe alkyle inférieur et R représente l'hydrogène; un groupe alkyle inférieur non substitué ou substitué par un groupe cycloalkyle contenant 3 à 7 atomes de carbone, un groupe hydroxy, alcoxy inférieur, carboxy ou carbalcoxy inférieur; un groupe cycloalkyle contenant 3 à 7 atomes de carbone; ou un alcanoyle inférieur et leur sels pharmaceutiquement acceptables.

Selon ce document, ces produits ont des propriétés pharmacologiques intéressantes, les composés ayant le substituent OR en position 7 de la tétraline ayant montré une activité lipolytique particulièrement prononcée.

Dans la présente description:
- le terme "alkyle inférieur", désigne un radical monovalent d'un hydrocarbure saturé contenant 1 à 4 atomes de carbone, tel que méthyle, éthyle, propyle, isopropyle ou n-butyle;
- le terme "carbalcoxy inférieur" désigne le groupe carboxyle, estérifié avec un alkyle inférieur tel que défini ci-dessus;
- le terme "halogène" comprend les quatre halogènes fluor, chlore, brome, iode, les trois premiers étant particulièrement préférés;
- les termes "tétraline" et "tétralone" se rapportent au 1,2,3,4-tétrahydronaphtalène.

Selon le brevet européen ci-dessus, les produits de formule (A) sont préparés selon divers modes opératoires qui comportent toujours la réaction d'une 2-aminotétraline ou d'une 2-oxotétraline avec
- une phényléthanolamine ou
- un époxyde de styrène ou
- un phénylglyoxal ou
- une alpha-haloacétophénone.

Parmi les méthodes de préparation des produits de formule (A) ci-dessus, est décrite une O-alkylation qui, à partir du 2-[(7-hydroxy-1,2,3,4-tétrahydronapht-2-yl)amino]-1-phényléthanol ou du 2-[(7-hydroxy-1,2,3,4-tétrahydronapht-2-yl)amino]-1-(3-chlorophényl)éthanol, par réaction avec du bromoacétate d'éthyle donne le 2-[(7-carbéthoxyméthoxy-1,2,3,4-tétrahydronapht-2-yl)amino]-1-phényléthanol, ci-après désigné COMPOSE 1, ou le 2-[7-carbéthoxyméthoxy-1,2,3,4-tétrahydronapht-2-yl)amino]-1-(3-chlorophényl)éthanol, ci-après désigné COMPOSE 2. Cette réaction ne donne cependant pas de bons résultats car le rendement en produit final est très faible.

Ces deux produits possèdent une activité sur la motilité intestinale très puissante et sélective, le COMPOSE 2 étant particulièrement intéressant (Digestive Diseases and Sciences, 1987, 32, 907).

La demande EP 253 257 indique, dans une formule générale, des aminotétralines de formule 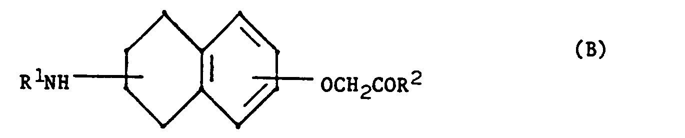
et, toujours dans une formule générale, des aminotétralines intermédiaires de formule 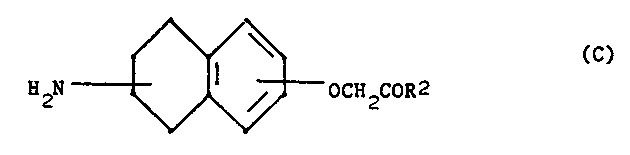
Dans les formules (B) et (C), R² représente, entre autres, OH ou OAlkyle.

Le document ci-dessus décrit toute une série d'O-ethers d'aminotétralines (C), mais non les dérivés ayant les substitutions dans les positions 2 et 7, qui, dans la série de produits de formule (A), notamment dans le cas du COMPOSE 1 et du COMPOSE 2, sont particulièrement intéressants.

On a maintenant trouvé qu'à partir d'une 2-amino-7-hydroxy-tétraline dans laquelle le groupe amino est protégé par un groupe susceptible d'être éliminé par hydrogénation catalytique ou par hydrolyse acide douce, il est possible, par O-alkylation et N-déprotection, de préparer la 2-amino-7-hydroxytétraline éthérifiée par un groupe méthyle substitué par un groupe carboxy ou par un groupe carbalcoxy inférieur.

On a également trouvé qu'en utilisant lesdites groupes protecteurs, il est possible de les éliminer, après la O-alkylation, sans hydrolyser les groupes carbalcoxy inférieur et d'obtenir ainsi l'aminotétraline libre correspondante.

On a enfin trouvé que la 2-amino-7-hydroxytétraline éthérifiée par un groupe méthyle substitué par un groupe carboxy ou carbalcoxy inférieur peut être utilisée pour préparer les phényléthanolaminotétralines correspondantes, notamment le COMPOSE 1 et le COMPOSE 2 ci-dessus, soit par réaction avec un époxyde de styrène, soit par réaction avec un acide mandélique et réduction du mandélamide obtenu.

La présente invention a donc pour objet, selon un de ses aspects, des éthers de la 2-amino-7-hydroxytétraline de formule 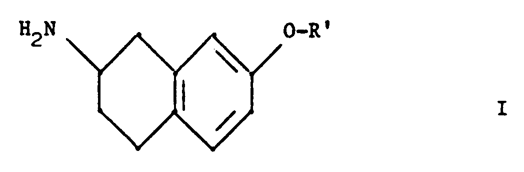
dans laquelle R′ représente un groupe méthyle substitué par un groupe carboxy ou carbalcoxy inférieur, et leurs sels.

Le groupe carbalcoxy inférieur préférentiel est le groupe carbéthoxy.

Selon un autre de ses aspects, la présente invention a pour objet un procédé pour la préparation des composés I et de leurs sels caractérisé en ce qu'on traite une 2-amino-7-hydroxytétraline N-protégée de formule 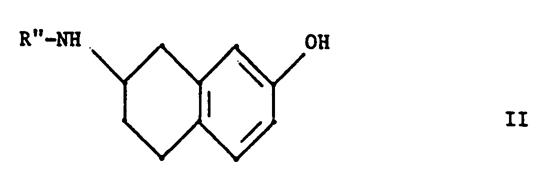
dans laquelle R˝ est un groupe N-protecteur susceptible d'être éliminé par hydrogénation catalytique ou par hydrolyse acide douce, avec un composé de formule

R′-Hal III

dans laquelle R′ est tel que défini ci-dessus et Hal est le chlore, le brome ou l'iode, en présence d'un agent de condensation basique, on soumet l'éther de 2-amino-7-hydroxytétraline N-protégé ainsi obtenu de formule 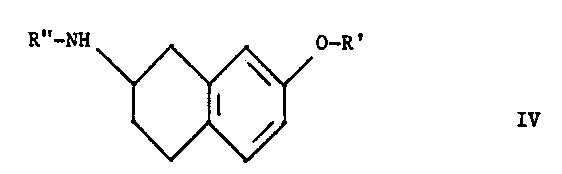
dans laquelle R′ et R˝ sont tels que définis ci-dessus, à une N-déprotection par hydrogénation catalytique ou par hydrolyse acide douce et, après avoir éventuellement saponifié le groupe carbalcoxy inférieur en groupe carboxy, on isole le produit de formule I éventuellement sous forme de sel et on le transforme éventuellement en l'un de ses sels.

Les groupes N-protecteurs R˝ préférentiels sont les groupes tert-butoxycarbonyle (Boc), benzyloxycarbonyle et, en général, les groupes N-protecteurs utilisés dans la chimie des peptides, ou les groupes benzyle, benzhydryle ou trityle, non substitués ou substitués par un groupe méthoxy sur le ou l'un des groupes phényle.

Comme composé de formule III, on utilise de préférence un bromoacétate d'alkyle inférieur, le bromoacétate de méthyle ou d'éthyle étant particulièrement préféré. La réaction est conduite dans un solvant organique tel que l'acétone, l'acétate d'éthyle ou le tétrahydrofuranne en utilisant un agent de condensation basique classique comme un carbonate alcalin, par exemple le carbonate potassique.

L'éther de 2-amino-7-hydroxytétraline N-protégé IV ainsi obtenu est isolé selon les techniques conventionnelles, éventuellement sous forme d'un de ses sels, et soumis à la N-déprotection.

L'élimination des groupes N-protecteurs est effectuée par hydrogénation catalytique ou par hydrolyse acide douce selon des méthodes bien connues de littérature. Notamment, le groupe Boc est éliminé en conditions acides, par action de l'acide trifluoroacétique ou chlorhydrique. Les autres sont tous éliminés par hydrogénation catalytique, de préférence en utilisant le palladium sur charbon comme catalyseur. Les groupes trityle et méthoxytrityle peuvent également être hydrolysés en conditions acides douces, par exemple par l'acide formique à 50% ou par l'acide chlorhydrique gazeux dans un solvant organique.

Les composés de formule I dans laquelle R′ représente méthyle substitué par un groupe carbalcoxy inférieur peuvent être soumis à une saponification pour obtenir le groupe carboxy correspondant, avant ou après la déprotection du groupe amino.

Les produits I sons isolés selon les méthodes conventionnelles, de préférence sous forme d'un de leurs sels. La base libre peut être libérée par neutralisation et transformée en un autre sel. Dans le cas du produit de formule I où R′ est méthyle substitué par un groupe carboxy, l'aminoacide obtenu peut être transformé en un sel d'addition acide ou en un sel avec un métal, notamment alcalin, tel que le sel de sodium.

La 2-amino-7-hydroxytétraline N-protégée de formule II est préparée soit à partir de la 2-amino-7-hydroxytétraline de formule IIa 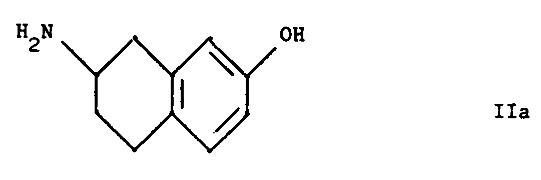
soit directement à partir de la 7-méthoxy-2-tétralone de formule V 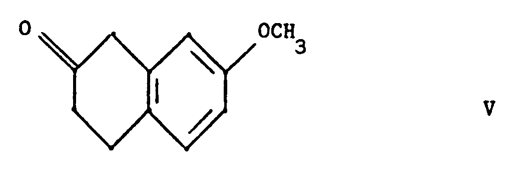
sans passer par la 2-amino-7-hydroxytétraline IIa.

La 2-amino-7-hydroxytétraline IIa est préparée à partir de la méthoxytétralone correspondante de formule V par réaction avec la benzylamine, réduction par du borohydrure de sodium de la benzylimine obtenue, débenzylation par hydrogénation catalytique et déméthylation par l'acide bromhydrique à 48%, selon le Schéma 1 suivant 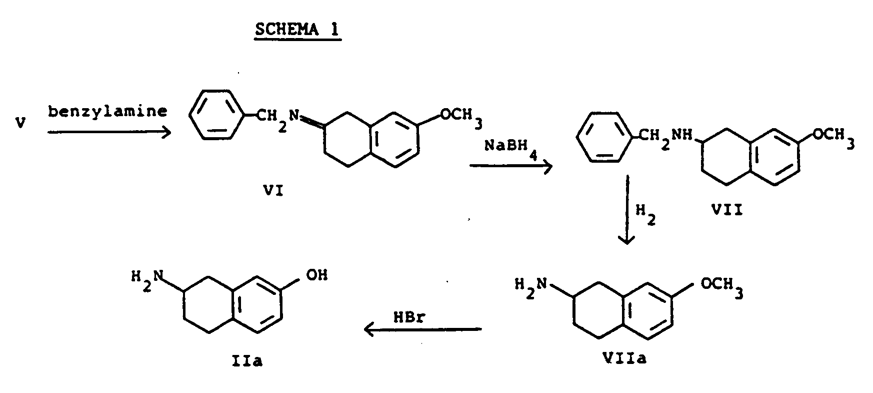
La réaction du produit V avec la benzylamine est effectuée selon les méthodes classiques de préparation des bases de Schiff, dans un solvant organique tel que le toluène en présence d'acide p-toluène-sulfonique et le composé VI ainsi obtenu peut être réduit, sans être isolé et purifié, avec le borohydrure de sodium. Par hydrogénation catalytique, en utilisant par exemple du palladium sur charbon, on obtient la 2-amino-7-méthoxytétraline VIIa qui, par chauffage dans de l'acide bromhydrique à 48%, donne la 2-amino-7-hydroxytétraline IIa, comme bromhydrate, transformé en la base libre par neutralisation.

Dans l'étape V → VI, la benzylamine peut être remplacée par la tritylamine ou la benzhydrylamine, les trois produits pouvant être substitués par un groupe méthoxy sur le ou l'un des groupes phényle.

Les deux formes optiquement actives des aminotétralines de formule IIa, VII et VIIa sont préparées par résolution du racémate selon des méthodes connues, par exemple par salification avec un acide optiquement actif, l'acide mandélique de préférence.

La N-protection par le group R˝ est effectuée en faisant réagir le composé de formule IIa avec le réactif approprié pour la protection des groupes amino comme décrit, par exemple, par M. Bodanszky et al., Peptide Synthesis, 2nd Edition, John Wiley & Sons 1976, pages 18 à 49, chapitres 3 à 6.

Le groupe Boc, par exemple, peut être introduit par réaction avec le di-tert-butyldicarbonate en milieu basique. Le groupe benzyloxycarbonyle peut être introduit selon la procédure générale décrite par E.C. Horning, Organic Synthesis, vol. III, Wiley, New York 1955, page 167.

Ainsi, par exemple, pour préparer les produits I, on peut protéger le groupe amino de la 2-amino-7-hydroxytétraline IIa par un groupe Boc par réaction avec di-tert-butyldicarbonate dans un solvant organique tel que le dioxane ou le diméthylformamide, traiter le produit ainsi obtenu avec un composé de formule III en milieu alcalin et déprotéger le groupe amino par élimination du groupe Boc avec de l'acide trifluoroacétique ou chlorhydrique selon le Schéma 2 suivant 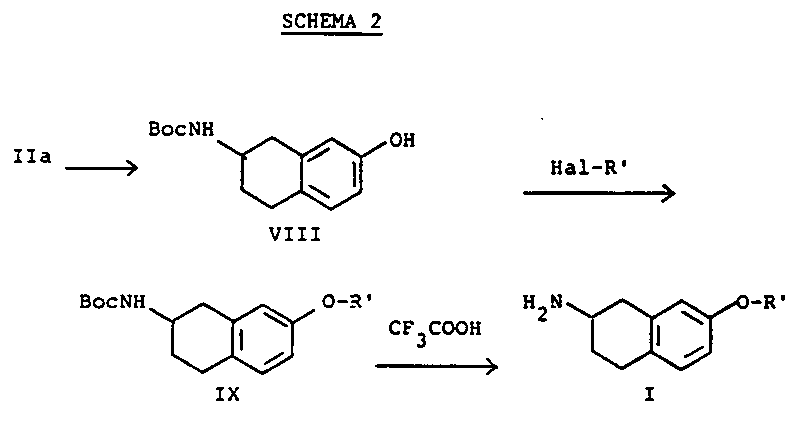
La libération de l'amine I par élimination du groupe N-protecteur Boc ne comporte pas de modification au niveau du groupe carbalcoxy inférieur ou de la stéréoconfiguration.

La N-protection peut également être effectuée à partir de la 7-méthoxy-2-tétralone V, sans passer par la 2-amino-7-hydroxytétraline IIa, par formation d'une base de Schiff avec une amine choisie parmi la benzylamine, la benzhydrylamine et la tritylamine, non substituées ou substituées par un groupe méthoxy sur le ou l'un des groupes phényle et réduction par le borohydrure de sodium.

Ainsi, par exemple, les produits I peuvent être préparés à partir de la 2-benzylamino-7-méthoxytétraline de formule VII (Schéma 1), par déméthylation avec de l'acide bromhydrique, réaction du phénol correspondant avec un composé de formule III en milieu alcalin et débenzylation, selon le Schéma 3 suivant 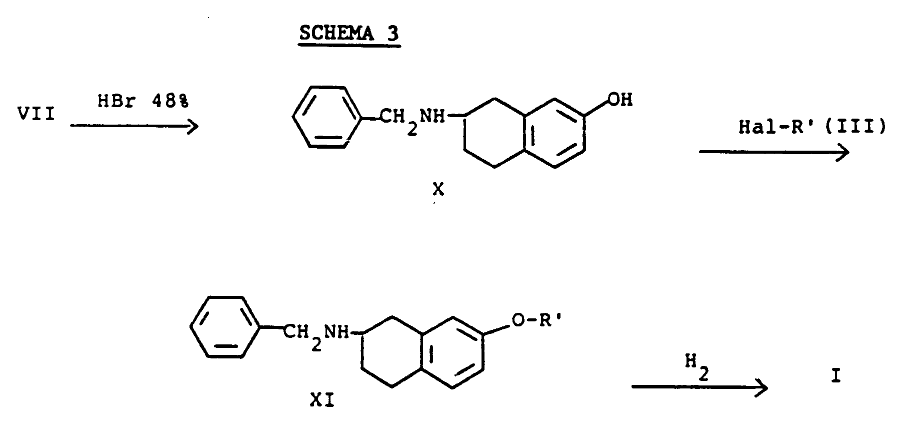
Dans le Schéma 2 ci-dessus, le groupe Boc peut être remplacé par le groupe benzyloxycarbonyle ou par tout autre groupe protecteur tel que défini ci-dessus. Dans le Schéma 3 le groupe benzyle peut être substitué sur le groupe phényle par un groupe méthoxy ou peut être remplacé par un groupe benzhydryle ou trityle non substitué ou substitué sur l'un des groupes phényle par un groupe méthoxy. La déprotection est effectuée comme indiqué ci-dessus.

Les éthers de la 2-amino-7-hydroxytétraline N-protégé IV et leurs sels éventuels sont de nouveaux intermédiaires, ceux dont le substituant R′ est (carbalcoxy inférieur)méthyle, par exemple carbométhoxyméthyle ou carbéthoxyméthyle, étant particulièrement préférés.

Ces produits, sous forme racémique ou sous forme de leurs stéréoisomères séparés, représentent un autre aspect de la présente invention.

Selon un aspect ultérieur, la présente invention a pour objet l'utilisation des éthers de la 2-amino-7-hydroxytétraline de formule I pour la préparation des phényléthanolaminotétralines correspondantes de formule XII 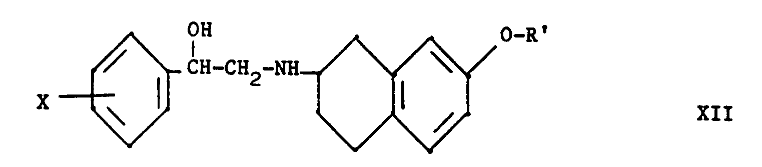
dans laquelle X et R′ sont tels que défini ci-dessus, et de leurs sels pharmaceutiquement acceptables.

Cette utilisation comporte soit une réaction des composés I avec un époxyde de styrène de formule 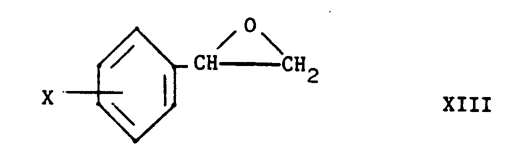
dans laquelle X est tel que défini ci-dessus, ledit époxyde de styrène pouvant être racémique ou, notamment lorsque X est hydrogène ou 3-chloro, optiquement actif, soit une réaction avec un dérivé fonctionnel d'un acide mandélique de formule 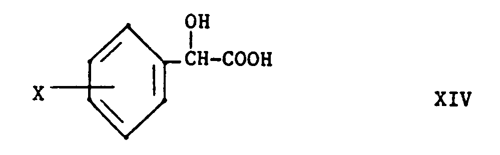
dans laquelle X est tel que défini ci-dessus et réduction du groupe carbonyle amidique du mandélamide ainsi obtenu de formule 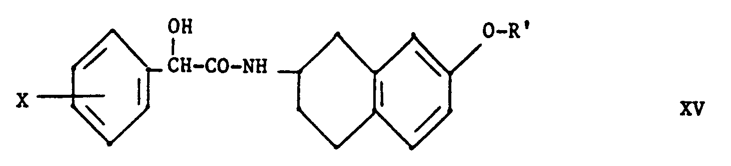
dans laquelle X et R′ sont tels que définis ci-dessus, en groupe méthylène, ledit acide mandélique XIV pouvant être racémique ou, notamment lorsque X est hydrogène ou 3-chloro, optiquement actif, et une salification éventuelle.

Les exemples suivants illustrent l'invention sans toutefois la limiter. Sauf indication spécifique, le symbole du pouvoir rotatoire est indiquée comme /alpha/, mais on doit l'entendre comme [alpha]_{D}²⁰.

### PREPARATION I

### Bromhydrate de 2-amino-7-hydroxytétraline.

(a) On chauffe au reflux pendant 3 heures un mélange de 8 g de 7-méthoxy-2-tétralone, 4,8 g de benzylamine, 150 ml de toluène anhydre et 100 mg d'acide p.toluènesulfonique. On évapore à sec, on reprend l'huile résiduelle par 100 ml de méthanol et à la solution obtenue on ajoute, avec précaution et à 0-5°C, 8,5 g de borohydrure de sodium. On laisse le mélange sous agitation et à la température ambiante pendant une nuit, puis on y ajoute 50 ml d'eau, on laisse sous agitation pendant 30 minutes, on évapore le solvant, on reprend avec 30 ml d'eau et 10 ml d'une solution concentrée d'hydroxyde d'ammonium. On extrait avec 200 ml d'acétate d'éthyle, on sèche la phase organique sur du sulfate de sodium, filtre et évapore à sec. On obtient une huile de couleur foncée qui est purifiée par flash chromatographie en utilisant comme éluant le mélange acétate d'éthyle/méthanol 95/5. On transforme la base obtenue en son chlorhydrate par dissolution dans 40 ml d'isopropanol et addition d'isopropanol saturé d'acide chlorhydrique gazeux. On obtient ainsi 11,4 g de chlorhydrate de 2-benzylamino-7-méthoxytétraline; p.f. 265-267°C (déc.).
(b) On soumet le produit ci-dessus, dissous dans 200 ml de méthanol et 100 ml d'eau, à une hydrogénation en présence de 1,2 g de palladium sur charbon à 10%, à la pression ambiante et à la température de 45-50°C. Après 4 heures on filtre, évapore à sec, reprend deux fois avec de l'éthanol absolu et l'on évapore à sec. On obtient un solide blanc qui est repris avec 70 ml d'isopropanol à chaud. Par refroidissement, la suspension obtenue précipite et donne 7,8 g de chlorhydrate de 2-amino-7-méthoxytétraline; p.f. 214-216°C.
(c) On met en suspension 6,6 g du produit ci-dessus dans 80 ml d'acide bromhydrique à 48% et on chauffe le mélange au reflux pendant 2 heures. On évapore à sec la solution obtenue, on reprend par de l'éthanol absolu et on évapore à sec deux fois. On obtient ainsi une huile qu'on dissout dans 20 ml d'isopropanol à chaud. Par addition de 30 ml d'éther éthylique à la solution on obtient 6,8 g de bromhydrate de 2-amino-7-hydroxytétraline cristallin; p.f. 171-173°C.

### PREPARATION II

### Monohydrate de (R)-2-amino-7-hydroxytétraline.

A une solution dans 550 ml d'éthanol absolu de 50 g de 2-amino-7-méthoxytétraline base brute, obtenue du chlorhydrate correspondant (PREPARATION I b) par neutralisation avec de l'hydroxyde de sodium à 10%, extraction avec de l'acétate d'éthyle et évaporation du solvant, on ajoute une solution de 43 g d'acide (+) mandélique dans 550 ml d'éthanol absolu. Après une nuit à la température ambiante, on filtre le précipité obtenu et on le cristallise deux fois dans l'éthanol absolu en recupérant chaque fois le produit cristallisé après repos d'une nuit à la température ambiante. On obtient ainsi 34,2 g (74%) de sel pur de l'acide (+) mandélique avec la (+)-2-amino-7-méthoxytétraline; p.f. 190-192°C. Les eaux mères de cette première cristallisation sont séparées et utilisées pour la PREPARATION III ci-dessous. On suspend 34 g du sel ainsi obtenu dans 300 ml d'eau et on rend basique le mélange réactionnel avec de l'hydroxyde de sodium N. On extrait la base avec de l'acétate d'éthyle, on évapore à sec et on reprend le residu par 260 ml d'acide bromhydrique à 48%. On chauffe au reflux le mélange réactionnel pendant trois heures, on l'évapore à sec sous vide et on reprend le résidu obtenu avec 70 ml d'eau. On rend basique la solution aqueuse avec de l'hydroxyde d'ammonium concentré, on la refroidit pendant une nuit et on la filtre. On obtient 17 g de (R)-2-amino-7-hydroxytétraline sous forme monohydratée; p.f. 143-144°C, /alpha/ = + 85,1° (méthanol, c = 0,5%).

Le chlorhydrate de ce produit a un pouvoir rotatoire qui correspond à celui de la littérature (Molecular Pharmacology, 1982, 22, 281-289).

### PREPARATION III

### Monohydrate de (S)-2-amino-7-hydroxytétraline.

On évapore à sec les eaux mères de la première cristallisation mises de côté dans la PREPARATION II, on suspend le résidu ainsi obtenu dans 300 ml d'eau et on rend la solution basique par de l'hydroxyde de sodium N. On extrait la base avec de l'acétate d'éthyle. Suivant le mode opératoire décrit dans la PREPARATION II et en utilisant comme produits de départ la base ainsi obtenue et l'acide (-) mandélique, on obtient le sel de l'acide (-) mandélique avec la (-)-2-amino-7-méthoxytétraline (p.f. 189-191°C) qui, par neutralisation et déméthylation avec HBr, donne 17 g de (S)-2-amino-7-hydroxy- tétraline, sous forme de monohydrate; p.f. 143-144°C, /alpha/ = -86,9° (méthanol, c = 0,5 %).
Le chlorhydrate de ce produit a un pouvoir rotatoire qui correspond à celui de la littérature (molecular Pharmacology 1982, 22, 281-289).

### PREPARATION IV

### Bromhydrate de (S)-2-benzylamino-7-hydroxytétraline.

(a) A une solution de 44 g de 2-benzylamino-7-méthoxytétraline base, obtenue par neutralisation du chlorhydrate décrit dans la PREPARATION I (a), dans 140 ml d'éthanol absolu on ajoute une solution de 24,5 g d'acide (-)-mandélique dans 150 ml d'éthanol absolu. Après une nuit à température ambiante, on filtre le sel cristallisé et on le lave à l'éther éthylique. On obtient 42 g de produit ayant un p.f. de 150-152°C et un 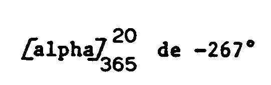 (méthanol, c = 1%) qui, après cristallisation, donne 33 g de sel (-)-mandélique; p.f. 155-157°C, 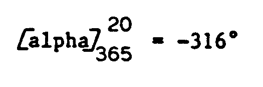 (méthanol, c = 1%).
   On neutralise 30 g du sel obtenu, dissous dans 400 ml d'eau, avec de l'hydroxyde d'ammonium à 32% et l'on extrait avec de l'acétate d'éthyle. On lave la phase organique à l'eau, on sèche sur du sulfate de sodium et l'on évapore. On obtient la (S)-2-benzylamino-7-méthoxytétraline base sous forme d'une huile qui est dissoute dans de l'isopropanol et traitée avec une solution d'HCl dans l'isopropanol. On filtre le chlorhydrate précipité et on le sèche. On obtient 22 g de produit; p.f. 287-290°C Par cristallisation de 1 g de produit dans un mélange méthanol/eau 1/1, on obtient 0,8 g de chlorhydrate de (S)-2-benzylamino-7-méthoxytétraline; p.f. 287-290°C, 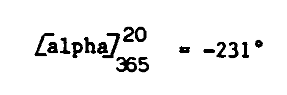 (méthanol, c = 1%).
(b) On chauffe au reflux une suspension de 15 g du produit ainsi obtenu dans 100 ml d'acide bromhydrique à 48% et 100 ml d'acide bromhydrique à 33% dans l'acide acétique, puis on évapore à sec la solution ainsi obtenue et on reprend et on évapore à sec trois fois avec de l'éthanol absolu. On dissout le résidu huileux dans de l'acétone et on cristallise. Ensuite on filtre le produit, on le lave d'abord avec de l'acétone et puis avec de l'éther éthylique et on sèche. On obtient ainsi 17 g de bromhydrate de (S)-2-benzylamino-7-hydroxytétraline; p.f. 185-187°C; 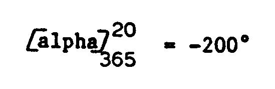 (méthanol, c = 1%).

### EXEMPLE 1

### Chlorhydrate de 2-benzylamino-7-carbéthoxyméthoxytétraline.

(a) On chauffe au reflux pendant 2 heures, sous agitation 25 g de chlorhydrate de 2-benzylamino-7-méthoxytétraline, PREPARATION I (a), dans 215 ml d'une solution à 33% d'acide bromhydrique dans l'acide acétique et en présence de 36 ml d'acide bromhydrique à 48%. Après concentration sous pression réduite, on reprend 3 fois le résidu avec 100 ml d'éthanol absolu en séchant chaque fois. On égrène le produit ainsi obtenu avec 150 ml d'acétone, on filtre et on obtient 25,3 g du bromhydrate de 2-benzylamino-7-hydroxytétraline; p.f. 198-200°C. On dissout dans 1300 ml d'eau chaude le sel et, après refroidissement, on ajoute de l'hydroxyde d'ammonium concentré. On extrait la base par l'acétate d'éthyle, on sèche et on obtient un solide qui est cristallisé dans 250 ml de toluène. On obtient 14 g de 2-benzylamino-7-hydroxytétraline base; p.f. 161-163°C.
(b) On chauffe à 70°C sous courant d'azote pendant 30 minutes 23 g de 2-benzylamino-7-hydroxytétraline base (a) et 4,5 g d'hydrure de sodium à 55% dans 800 ml de toluène. On ajoute goutte à goutte à la température ambiante un mélange de 15,2 g de bromoacétate d'éthyle et de 0,5 g de bromure de tétrabutylammonium dans 200 ml de toluène, puis on chauffe le mélange réactionnel 3 heures à 70%C, on refroidit, on ajoute 100 ml d'eau et on sépare la phase organique. On lave à l'eau, on sèche et on concentre. On obtient 31 g d'une huile qui est solubilisée dans l'isopropanol. Après avoir ajouté une solution d'isopropanol saturé d'acide chlorhydrique gazeux, on obtient 28 g du chlorhydrate de 2-benzylamino-7-carbéthoxyméthoxytétraline; p.f. 188-190°C.

### EXEMPLE 2

### Chlorhydrate de 2-amino-7-carbéthoxyméthoxytétraline.

On soumet une solution de 27 g de chlorhydrate de 2-benzylamino-7-carbéthoxyméthoxytétraline, EXEMPLE 1, dans 300 ml d'éthanol 95% et 25 ml d'eau, à hydrogénation à la pression ambiante et à la température de 50°C en utilisant comme catalysateur 3 g de palladium sur charbon à 10%. Après 3 heures, on filtre et on reprend le résidu 2 fois avec 100 ml d'éthanol absolu en séchant chaque fois. On égraine le produit obtenu dans 150 ml d'acétone, on filtre et on cristallise dans 100 ml d'isopropanol. On obtient 19 g du chlorhydrate de 2-amino-7-carbéthoxyméthoxytétraline; p.f. 143-145°C.
Le produit ainsi obtenu peut être utilisé pour la préparation du composé de formule (XV) ci-dessus, où X est l'hydrogène et O-R′ est un groupe carbéthoxyméthoxy (COMPOSE 1), de la façon suivante: On Chauffe au reflux pendant 6 heures une solution de 1,8 g de phényloxirane et 3,7 g de 2-amino-7-carbéthoxyméthoxytétraline base, obtenue par néutralisation du chlorhydrate décrit ci-dessus dans 40 ml de n-butanol. Après avoir concentré à sec, on purifie le résidu par flash chromatographie, en éluant avec un mélange acétate d'éthyle/méthanol 9/1. On laisse réagir pendant une nuit l'huile purifiée avec un excès d'acide oxalique dans 5 ml d'isopropanol. On obtient ainsi 1,9 g d'oxalate de N-(7-carbéthoxyméthoxy-1,2,3,4-tétrahydronapht-2-yl)-2-hydroxy-2-phényléthanamine; p.f. 159-162°C, identique au composé de l'Exemple 5 du brevet européen 211 721.

### EXEMPLE 3

### (R)-2-tert-butoxycarbonylamino-7-carbéthoxyméthoxytétraline.

(a) On chauffe à 40°C sous agitation une solution de 5,4 g de monohydrate de (R)-2-amino-7-hydroxytétraline, PREPARATION II, dans 40 ml de diméthylformamide en présence de 15 g de triéthylamine. On refroidit à 20°C le mélange réactionnel ainsi obtenu et on ajoute 7,2 g de di-tert-butyldicarbonate. On laisse le mélange réactionnel sous agitation pendant 3 heures à la température ambiante, puis on ajoute 100 ml d'eau et l'on extrait avec 240 ml d'éther éthylique. La phase organique est lavée à l'eau, séchée sur du sulfate de sodium et évaporée à sec. On obtient 10,8 g de (R)-2-tert-butoxycarbonylamino-7-hydroxytétraline sous forme d'une huile.
(b) On dissout 10,8 g de (R)-2-tert-butoxycarbonylamino-7-hydroxytétraline (a) dans 300 ml d'acétone, puis on chauffe la solution ainsi obtenue au reflux sous agitation pendant 6 heures en présence de 12,4 g de carbonate de potassium anhydre en poudre et 15,1 g de bromoacétate d'éthyle. On filtre, on évapore l'acétone sous pression réduite et on reprend avec de l'éther éthylique. On lave à l'eau la solution ainsi obtenue, on la sèche sur du sulfate de sodium et on l'évapore à sec. On reprend le résidu avec de l'éther éthylique et on cristallise dans de l'éther isopropylique. On obtient ainsi 4,8 g de (R)-2-tert-butoxycarbonylamino-7-carbéthoxyméthoxytétraline; p.f. 113-115°C, /alpha/ = + 57,97° (méthanol, c = 1%).

### EXEMPLE 4

### Chlorhydrate de (R)-2-amino-7-carbéthoxyméthoxytétraline.

(a) On dissout 3,5 g de (R)-2-tert-butoxycarbonylamino-7-carbéthoxyméthoxytétraline, EXEMPLE 3, dans 35 ml de chlorure de méthylène et on refroidit la solution ainsi obtenue à la température de 0°C. On ajoute une solution de 7,7 ml d'acide trifluoroacétique dans 40 ml de chlorure de méthylène et on laisse le mélange réactionnel sous agitation d'abord 30 minutes à la température de 0-5°C et ensuite 4 heures à la température ambiante. On neutralise avec une solution de bicarbonate de sodium et on sépare la phase organique qui est ensuite lavée à l'eau, séchée et évaporée à sec. On reprend le résidu avec 15 ml d'acétate d'éthyle et on le rend acide avec une solution d'acide chlorhydrique dans l'éthanol. On obtient un précipité qui est filtré et lavé avec de l'éther. On obtient 1,1 g du chlorhydrate de (R)-2-amino-7-carbéthoxyméthoxytétraline; p.f. 168-171°C, /alpha/ = +51,14° (méthanol, c = 1%).
(b) On dissout à la température ambiante 5,8 g de (R)-2-tert-butoxycarbonylamino-7-carbéthoxyméthoxytétraline, EXEMPLE 3, dans 135 ml d'éthanol absolu et à la solution ainsi obtenue on ajoute 82 ml d'une solution 7,2 N d'acide chlorhydrique gazeux dans l'éthanol. On laisse le mélange réactionnel sous agitation pendant 150 minutes à la température ambiante, puis on évapore à sec sous pression réduite. On reprend le résidu avec de l'éther éthylique et l'on filtre. On obtient ainsi 4,6 g de chlorhydrate de (R)-2-amino-7-carbéthoxyméthoxytétraline; p.f. 170-171°C, /alpha/ = +51° (méthanol, c = 1%). Par cristallisation dans l'éthanol, le p.f. reste inchangé (170-172°C) et la valeur de /alpha/ monte à + 51,6° (méthanol, c = 1%).

### EXEMPLE 5

### (S)-2-tert-butoxycarbonylamino-7-carbéthoxyméthoxytétraline.

(a) On chauffe à 40°C sous agitation une solution de 5,4 g de monohydrate de (S)-2-amino-7-hydroxytétraline, PREPARATION III, dans 40 ml de diméthylformamide en présence de 15 g de triéthylamine. On refroidit à 20°C le mélange réactionnel ainsi obtenu et on ajoute 7,2 g de di-tert-butyldicarbonate. En opérant comme décrit dans l'EXEMPLE 3(a), on obtient la (S)-2-tert-butoxycarbonylamino-7-hydroxytétraline qui est utilisée directement pour l'étape suivante.
(b) On dissout la (S)-2-tert-butoxycarbonylamino-7-hydroxytétraline obtenue dans (a) dans 300 ml d'acétone, et on opère ensuite comme décrit dans l'EXEMPLE 3(b). On obtient ainsi la (S)-2-tert-butoxycarbonylamino-7-carbéthoxyméthoxytétraline; /alpha/ = -59° (méthanol, c = 1%).

### EXEMPLE 6

### Chlorhydrate de (S)-2-amino-7-carbéthoxyméthoxytétraline.

On dissout 1 g de (S)-2-[N-(tert-butoxycarbonyl)amino]-7-carbéthoxyméthoxytétraline, EXEMPLE 5, dans 15 ml de chlorure de méthylène et on refroidit la solution ainsi obtenue à la température de 0°C. On ajoute une solution de 2,2 ml d'acide trifluoroacétique dans 15 ml de chlorure de méthylène et on laisse le mélange réactionnel sous agitation d'abord 30 minutes à la température de 0-5°C et ensuite 4 heures à la température ambiante. En opérant comme décrit dans l'EXEMPLE 4, on obtient le chlorhydrate de (S)-2-amino-7-carbéthoxyméthoxytétraline; /alpha/ = -52° (méthanol, c = 1%).

Le produit ainsi obtenu peut être utilisé pour la préparation de la N-[(2S)-7-carbéthoxyméthoxy-1,2,3,4-tétrahydronapht-2-yl]-(2R)-2-hydroxy-2-phényléthanamine (isomère R,S du COMPOSE 1) de la façon suivante:
(a) A une suspension de 5,7 g du chlorhydrate de (S)-2-amino-7-carbéthoxyméthoxytétraline, 3 g d'acide (R)-mandélique et 8 g d'hexafluorophosphate de benzotriazolyl-N-oxytris(diméthylamino)phosphonium (BOP) dans 100 ml de chlorure de méthylène anhydre, on ajoute lentement 5,6 ml de triéthylamine, puis on laisse la solution obtenue sous agitation à la température ambiante pendant 5 heures. On ajoute 150 ml d'une solution saturée de chlorure de sodium, on laisse sous agitation pendant 30 minutes à la température ambiante, on ajoute 500 ml d'acétate d'éthyle et on lave la phase organique d'abord avec une solution d'acide chlorhydrique 1N puis avec une solution saturée de bicarbonate de sodium et chlorure de sodium. On sèche la solution organique sur du sulfate de sodium, on la filtre et on l'évapore à sec. On purifie l'huile obtenue par flash chromatographie en utilisant comme solvant un mélange acétate d'éthyle/cyclohexane 55/45. On obtient un solide qu'on égrène dans 20 ml d'éther éthylique. On cristallise le produit obtenu dans l'acétate d'éthyle. On obtient le N-[(2S)-7-carbéthoxyméthoxy-1,2,3,4-tétrahydronapht-2-yl]-(R)-mandélamide; p.f. 115-117°C; /alpha/ = -98,3° (méthanol, c = 1%). Rendement: 55%.
(b) A une solution de 5 g de N-[(2S)-7-carbéthoxyméthoxy-1,2,3,4-tétrahydronapht-2-yl]-(R)-mandélamide dans 50 ml de tétrahydrofuranne anhydre, on ajoute goutte à goutte pendant 10 minutes à la température de 0°C sous courant d'azote 2,6 ml d'une solution 10 M de borane-méthylsulfure (réactif générant le diborane constitué par le complexe entre le borane et le diméthylsulfure) dans 10 ml de tétrahydrofuranne anhydre et on garde le mélange réactionnel pendant une nuit à la température ambiante. On détruit le borane-méthylsulfure qui n'a pas réagi par addition, avec précaution, de 30 ml d'éthanol absolu et chauffage au reflux pendant 30 minutes. On évapore à sec et on opère une flash chromatographie en éluant avec un mélange acétate d'éthyle/ méthanol 85/15. On sépare deux produits qu'on reprend par de l'éther éthylique. Le produit moins polaire donne, après cristallisation dans l'acétate d'éthyle, 1,2 g de N-[(2S)-7-carbéthoxyméthoxy-1,2,3,4-tétrahydronapht-2-yl]-(2R)-2-hydroxy-2-phényléthanamine; p.f. 108-111°C; /alpha/ = -78,65° (méthanol, c = 1%). Le produit plus polaire donne, après cristallisation dans l'acétate d'éthyle, 0,5 g de N-[(2S)-7-(2-hydroxyéthoxy-1,2,3,4-tétrahydronapht-2-yl]-(2R)-2-hydroxy-2-phényléthanamine; p.f. 94-96°C; /alpha/ = -83,68° (méthanol, c = 1%).

## Revendications (Revendications pour l'(les) Etat(s) contractant(s) suivant(s): AT, BE, CH, DE, FR, GB, GR, IT, LI, LU, NL, SE)

1. Ether de la 2-amino-7-hydroxytétraline de formule : 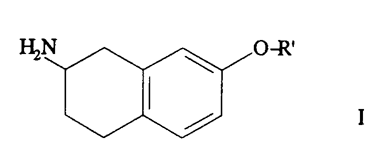 dans laquelle R' représente un groupe méthyle substitué par un groupe carboxy ou carbalcoxy inférieur ou un de ses sels.

2. Composé selon la revendication 1, caractérisé en ce qu'il est le 2-amino-7-carbéthoxyméthoxy-1,2,3,4-tétrahydronaphtalène ou un de ses sels.

3. Composé selon la revendication 1, caractérisé en ce qu'il est le (R)-2-amino-7-carbéthoxyméthoxy-1,2,3,4-tétrahydronaphtalène ou un de ses sels.

4. Composé selon la revendication 1, caractérisé en ce qu'il est le (S)-2-amino-7-carbéthoxyméthoxy-1,2,3,4-tétrahydronaphtalène ou un de ses sels.

5. Procédé pour la préparation d'éthers de la 2-amino-7-hydroxytétraline de la revendication 1, caractérisé en ce qu'on traite une 2-amino-7-hydroxytétraline N-protégée de formule : 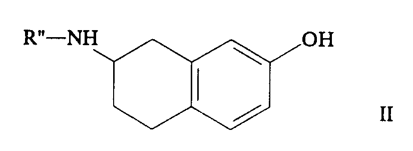 dans laquelle R'' est un groupe N-protecteur susceptible d'être éliminé par hydrogénation catalytique ou par hydrolyse acide douce, avec un composé de formule :
R'-Hal III
dans laquelle R' est tel que défini dans la revendication 1 et Hal représente chlore, brome ou iode, en présence d'un agent de condensation basique, on soumet l'éther de la 2-amino-7-hydroxytétraline N-protégé ainsi obtenu de formule : 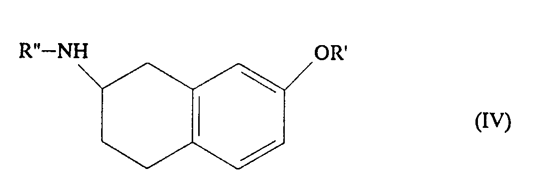 dans laquelle R' et R'' sont tels que définis ci-dessus, à une N-déprotection par hydrogénation catalytique ou par hydrolyse acide douce et, après avoir éventuellement saponifié le groupe carbalcoxy inférieur en groupe carboxy, on isole le produit final, éventuellement sous forme de sel et on le transforme éventuellement dans un de ses sels.

6. Procédé selon la revendication 5, caractérisé en ce que le groupe N-protecteur est choisi parmi les groupes tert-butoxycarbonyle, benzyloxycarbonyle, et les groupes benzyle, benzhydryle et trityle, non substitués ou substitués par un groupe méthoxy sur le ou l'un des groupes phényle.

7. Ether de la 2-amino-7-hydroxytétraline N-protégée de formule : 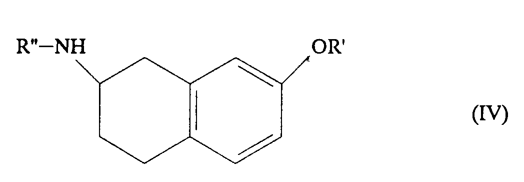 dans laqulle R' est tel que défini dans la revendication 1 et R'' est tel que défini dans la revendication 5, ou un de ses sels.

8. Composé de la revendication 7, caractérisé en ce que R'' est choisi parmi les groupes t-butoxycarbonyle, benzyloxycarbonyle, et les groupes benzyle, benzhydryle et trityle, non substitués ou substitués par un groupe méthoxy sur le cycle phényle.

9. Composé selon la revendication 8, caractérisé en ce qu'il est le 2-benzylamino-7-carbéthoxyméthoxy-1,2,3,4-tétrahydronaphtalène ou un de ses sels.

10. Composé selon la revendication 8, caractérisé en ce qu'il est le 2-tert-butoxycarbonylamino-7-carbéthoxyméthoxy-1,2,3,4-tétrahydronaphtalène.

11. Composé selon la revendication 8, caractérisé en ce qu'il est le (R)-2-tert-butoxycarbonylamino-7-carbéthoxyméthoxy-1,2,3,4-tétrahydronaphtalène.

12. Composé selon la revendication 8, caractérisé en ce qu'il est le (S)-2-tert-butoxycarbonylamino-7-carbéthoxyméthoxy-1,2,3,4-tétrahydronaphtalène.

13. Utilisation d'un éther de 2-amino-7-hydroxytétraline selon l'une quelconque des revendications 1 à 4, pour la préparation de la phényléthanolaminotétraline correspondante de formule : 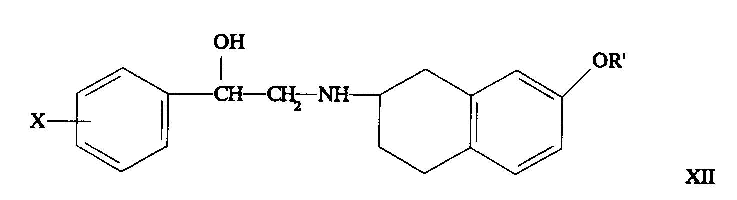 dans laquelle X représente l'hydrogène, un halogène, un groupe trifluorométhyle ou un groupe alkyle inférieur et R' est tel que défini dans la revendication 1, soit par réaction avec un époxyde de styrène de formule : 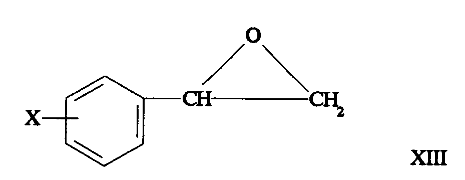 où X est tel que défini ci-dessus, soit par réaction avec un dérivé fonctionnel d'un acide mandélique de formule : 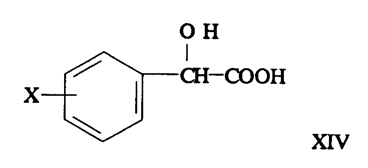 dans laquelle X est tel que défini ci-dessus et réduction du groupe carbonyle amidique du mandélamide ainsi obtenu de formule : 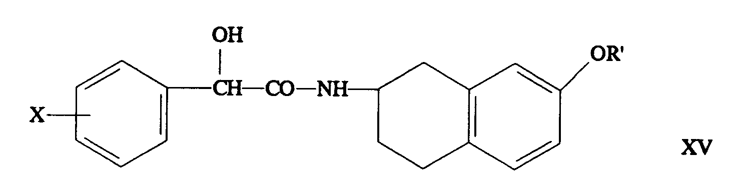 dans laquelle X et R' sont tels que définis ci-dessus, en groupe méthylène, et une salification éventuelle.

## Revendications (Revendications pour l'(les) Etat(s) contractant(s) suivant(s): ES)

1. Procédé pour la préparation d'éthers de la 2-amino-7-hydroxytétraline de formule : 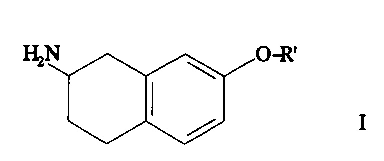 dans laquelle R' représente un groupe méthyle substitué par un groupe carboxy ou carbalcoxy inférieur, ou un de ses sels, caractérisé en ce qu'on traite une 2-amino-7-hydroxytétraline N-protégée de formule : 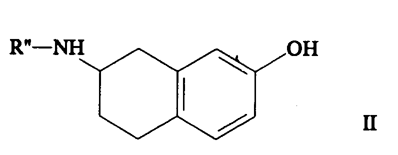 dans laquelle R'' est un groupe N-protecteur susceptible d'être éliminé par hydrogénation catalytique ou par hydrolyse acide douce, avec un composé de formule :
R'-Hal III
dans laquelle R' est tel que défini ci-dessus et Hal représente chlore, brome ou iode, en présence d'un agent de condensation basique, on soumet l'éther de la 2-amino-7-hydroxy-tétraline N-protégé ainsi obtenu de formule : 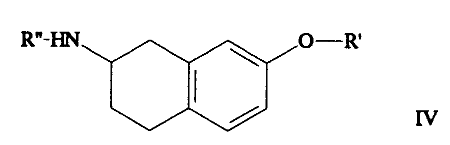 dans laquelle R' et R'' sont tels que définis ci-dessus, à une N-déprotection par hydrogénation catalytique ou par hydrolyse acide douce et, après avoir éventuellement saponifié le groupe carbalcoxy inférieur en groupe carboxy, on isole le produit final, éventuellement sous forme de sel et on le transforme éventuellement dans un de ses sels.

2. Procédé selon la revendication 1, caractérisé en ce que le groupe N-protecteur est choisi parmi les groupes tert-butoxycarbonyle, benzyloxycarbonyle, et les groupes benzyle, benzhydryle et trityle, non substitués ou substitués par un groupe méthoxy sur le ou l'un des groupes phényle.

3. Procédé selon la revendication 1 pour la préparation d'un composé de formule I dans laquelle R' est un groupe carbéthoxyméthoxy.

4. Procédé selon la revendication 1, pour la préparation d'un composé de formule I dans laquelle R' est un groupe carbéthoxyméthoxy et l'atome de carbone qui porte le groupe amino a la configuration absolue (R).

5. Procédé selon la revendication 1 pour la préparation d'un composé de formule I dans laquelle R' est un groupe carbéthoxyméthoxy et l'atome de carbone qui porte le groupe amino a la configuration absolue (S).

6. Procédé pour la préparation d'éthers de la 2-amino-7-hydroxytétraline N-protégée de formule IV : 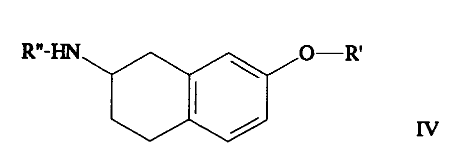 dans laquelle R' et R'' sont tels que définis dans la revendication 1, ou d'un de ses sels, caractérisé en ce qu'on traite une 2-amino-7-hydroxytétraline N-protégée de formule II : 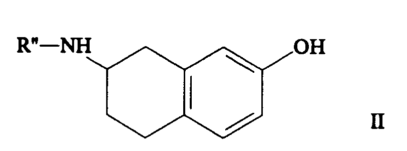 dans laquelle R'' est tel que défini ci-dessus, avec un composé de formule III
R' - Hal III
dans laquelle R' est tel que défini ci-dessus et Hal représente chlore, brome ou iode, en présence d'un agent de condensation basique, et on isole le composé de formule IV éventuellement sous forme d'un de ses sels, selon les techniques conventionnelles.

7. Procédé selon la revendication 6, caractérisé en ce que R'' est choisi parmi les groupes t-butoxycarbonyle, benzyloxycarbonyle, et les groupes benzyle, benzhydryle et trityle, non substitués ou substitués par un groupe méthoxy sur le cycle phényle.

8. Procédé selon la revendication 7 caractérisé en ce que R' est un groupe carboéthoxyméthyle et R'' est choisi parmi les groupes benzyle et t-butoxycarbonyle.

9. Procédé selon la revendication 7, caractérisé en ce que R' est un groupe carboéthoxyméthyle, R'' est un groupe t-butoxycarbonyle et l'atome de carbone qui porte le groupe -NHR'' à la configuration absolue (R) ou (S).

10. Utilisation d'un éther de la 2-amino-7-hydroxytétraline préparée par le procédé selon l'une quelconque des revendications 1 à 9 pour la préparation de la phényléthanolaminotétraline correspondante de formule : 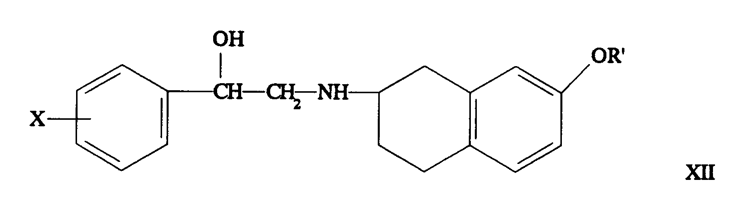 dans laquelle X représente l'hydrogène, un halogène, un groupe trifluorométhyle ou un groupe alkyle inférieur et R' est tel que défini dans la revendication 1, soit par réaction avec un époxyde de styrène de formule : 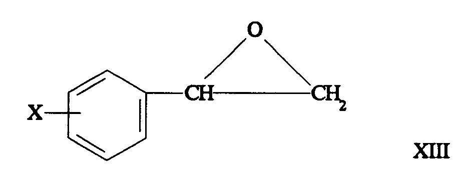 où X est tel que défini ci-dessus, soit par réaction avec un dérivé fonctionnel d'un acide mandélique de formule : 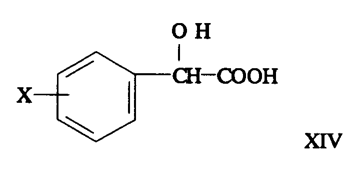 dans laquelle X est tel que défini ci-dessus et réduction du groupe carbonyle amidique du mandélamide ainsi obtenu de formule : 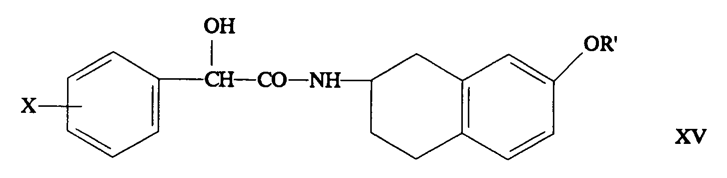 dans laquelle X et R' sont tels que définis ci-dessus, en groupe méthylène, et une salification éventuelle.

## Claims (Claims for the following Contracting State(s): AT, BE, CH, DE, FR, GB GR, IT, LI, LU, NL, SE)

1. 2-amino-7-hydroxytetraline ether of formula: 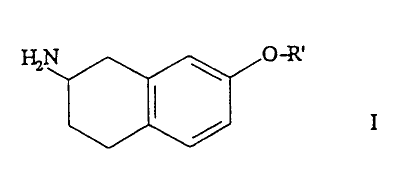 in which R' represents a methyl group substituted by a carboxy or lower carbalkoxy group, or a salt thereof.

2. Compound according to claim 1, characterized in that it is the 2-amino-7-carbethoxymethoxy-1,2,3,4-tetrahydronaphthalene or a salt thereof.

3. Compound according to claim 1, characterized in that it is the (R)-2-amino-7-carbethoxymethoxy-1,2,3,4-tetrahydronaphthalene or a salt thereof.

4. Compound according to claim 1, characterized in that it is the (S)-2-amino-7-carbethoxymethoxy-1,2,3,4-tetrahydronaphthalene or a salt thereof.

5. Process for the preparation of 2-amino-7-hydroxytetraline ethers of claim 1, characterized in that a N-protected 2-amino-7-hydroxytetraline of formula 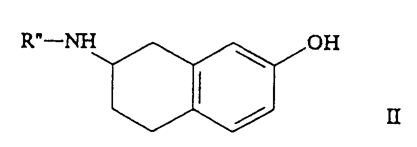 in which R'' is a N-protecting group liable of being eliminated by catalytic hydrogenation or by mild acid hydrolysis, is treated with a compound of formula:
R'-Hal III
in which R' is as defined in claim 1 and Hal is chlorine, bromine or iodine, in the presence of a basic condensing agent, the N-protected 2-amino-7-hydroxytetraline ether thus obtained of formula 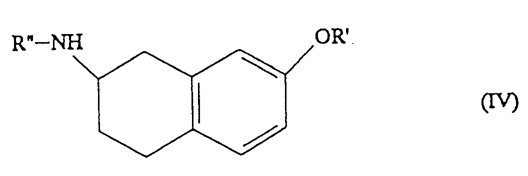 in which R' and R'' are as defined above, is submitted to a N-deprotection by catalytic hydrogenation or by mild acid hydrolysis and, after having, if desired, transformed the lower carbalkoxy group into a carboxy group by saponification, the final product is possibly isolated in the form of a salt, and, if desired, it is transformed into one of its salts.

6. Process according to claim 5, characterized in that the N-protecting group is selected from the groups consisting of tert-butoxycarbonyl, benzyloxycarbonyl and the benzyl, benzhydryl and trityl groups, unsubstituted or substituted by a methoxy group on the phenyl group or one of the phenyl groups.

7. N-protected 2-amino-7-hydroxytetraline ether of formula: 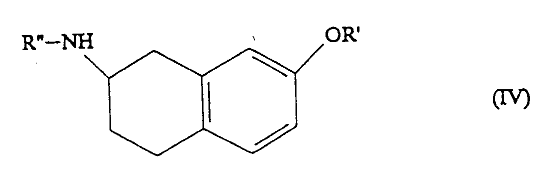 in which R' is as defined in claim 1 and R'' is as defined in claim 5, or a salt thereof.

8. Compound according to claim 7, characterized in that R'' is selected from the groups consisting of t-butoxycarbonyl, benzyloxycarbonyl and the benzyl, benzhydryl and trityl groups unsubstituted or substituted by a methoxy group in a phenyl ring.

9. Compound according to claim 8, characterized in that it is the 2-benzylamino-7-carbethoxymethoxy-1,2,3,4-tetrahydronaphthalene or a salt thereof.

10. Compound according to claim 8, characterized in that it is the 2-tert-butoxycarbonylamino-7-carbethoxymethoxy-1,2,3,4-tetrahydronaphthalene.

11. Compound according to claim 8, characterized in that it is the (R)-2-tert-butoxycarbonylamino-7-carbethoxymethoxy-1,2,3,4-tetrahydronaphthalene.

12. Compound according to claim 8, characterized in that it is the (S)-2-tert-butoxycarbonylamino-7-carbethoxymethoxy-1,2,3,4-tetrahydronaphthalene.

13. Use of a 2-amino-7-hydroxytetraline ether according to any one of claims 1 to 4, for the preparation of the corresponding phenylethanolaminotetraline of formula: 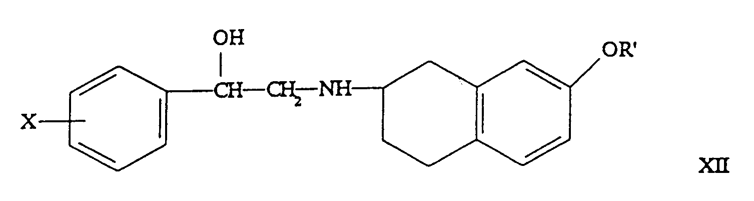 in which X is hydrogen, a halogen, a trifluoromethyl group or a lower alkyl group and R' is as defined in claim 1, either by a reaction with a styrene epoxide of formula: 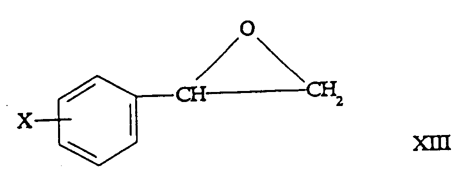 where X is as defined above, or by a reaction with a functional derivative of a mandelic acid of formula: 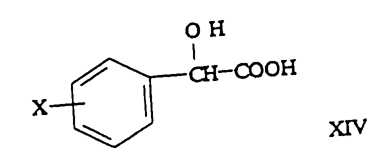 in which X is as defined above and a reduction of the amidic carbonyl group of the mandelamide thus obtained of formula 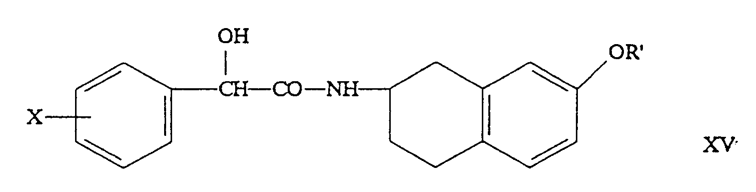 in which X and R' are as defined above, to a methylene group, and a possible salification.

## Claims (Claims for the following Contracting State(s): ES)

1. Process for the preparation of 2-amino-7-hydroxytetraline ethers of formula: 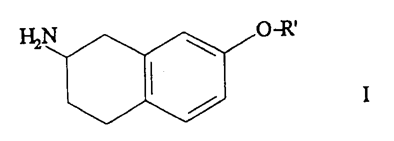 in which R' represents a methyl group substituted by a carboxy or lower carbalkoxy group, or a salt thereof, characterized in that a N-protected 2-amino-7-hydroxytetraline of formula 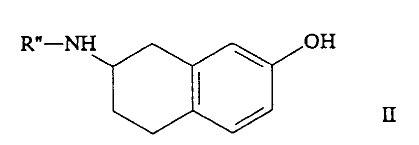 in which R'' is a N-protecting group liable of being eliminated by catalytic hydrogenation or by mild acid hydrolysis, is treated with a compound of formula:
R'-Hal III
in which R' is as defined above and Hal is chlorine, bromine or iodine, in the presence of a basic condensing agent, the N-protected 2-amino-7-hydroxytetraline ether thus obtained of formula 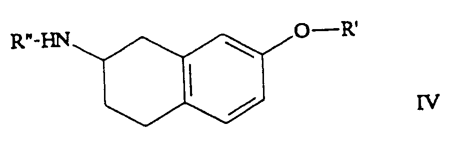 in which R' and R'' are as defined above, is submitted to a N-deprotection by catalytic hydrogenation or by mild acid hydrolysis and, after having, if desired, transformed the lower carbalkoxy group into carboxy group by saponification, the final product is possibly isolated in the form of a salt, and, if desired, it is transformed into one of its salts.

2. Process according to claim 1, characterized in that the N-protecting group is selected from the groups consisting of tert-butoxycarbonyl, benzyloxycarbonyl and the benzyl, benzhydryl and trityl groups, unsubstituted or substituted by a methoxy group on the phenyl group or one of the phenyl groups.

3. Process according to claim 1 for the preparation of a compound of formula I in which R' is a carbethoxymethoxy group.

4. Process according to claim 1 for the preparation of a compound of formula I in which R' is a carbethoxymethoxy group and the carbon atom carrying the amino group has the absolute (R) configuration.

5. Process according to claim 1 for the preparation of a compound of formula I in which R' is a carbethoxymethoxy group and the carbon atom carrying the amino group has the absolute (S) configuration.

6. Process for the preparation of N-protected 2-amino-7-hydroxytetraline ethers of formula IV: 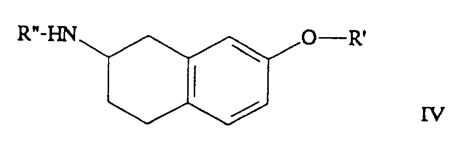 in which R' and R'' are as defined in claim 1, or a salt thereof, characterized in that a N-protected 2-amino-7-hydroxytetraline of formula II 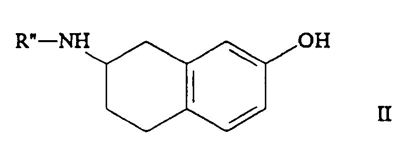 in which R'' is as defined above, is treated with a compound of formula III
R'-Hal III
in which R' is as defined above and Hal is chlorine, bromine or iodine, in the presence of a basic condensing agent, and the compound of formula IV is possibly isolated in the form of a salt thereof according to the conventional techniques.

7. Process according to claim 6, characterized in that R'' is selected from the groups consisting of t-butoxycarbonyl, benzyloxycarbonyl and benzyl, benzhydryl and trityl groups, unsubstituted or substituted by a methoxy group in a phenyl ring.

8. Process according to claim 7, characterized in that R' is a carboethoxymethyl group and R'' is selected from the benzyl and t-butoxycarbonyl groups.

9. Process according to claim 7, characterized in that R' is a carboethoxymethyl group, R'' is a t-butoxycarbonyl group and the carbon atom carrying the -NHR'' group has the absolute (R) or (S) configuration.

10. Use of a 2-amino-7-hydroxytetraline ether prepared by the process according to any one of claims 1 to 9, for the preparation of the corresponding phenylethanolaminotetraline of formula: 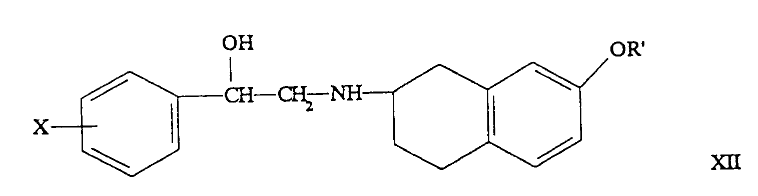 in which X is hydrogen, a halogen, a trifluoromethyl group or a lower alkyl group and R' is as defined in claim 1, either by reaction with a styrene epoxide of formula: 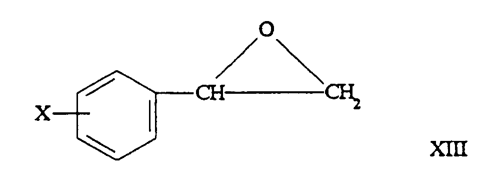 where X is as defined above, or by reaction with a functional derivative of a mandelic acid of formula: 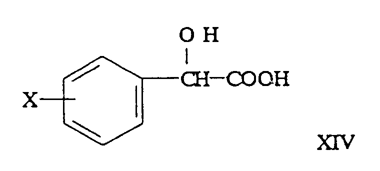 in which X is as defined above and a reduction of the amidic carbonyl group of the mandelamide thus obtained of formula: 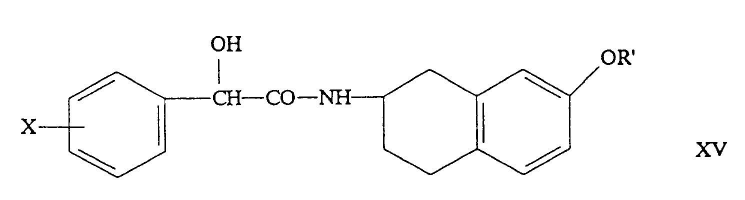 in which X and R' are as defined above, to a methylene group, and a possible salification.

## Patentansprüche (Patentansprüche für folgende(n) Vertragsstaat(en): AT, BE, CH, DE, FR, GB, GR, IT, LI, LU, NL, SE)

1. 2-Amino-7-hydroxytetralinether der Formel 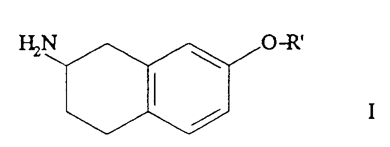 worin R' eine durch eine Carboxy- oder nied.Carbalcoxygruppe substituierte Methylgruppe darstellt, oder eines seiner Salze.

2. Verbindung nach Anspruch 1, dadurch gekennzeichnet, daß sie 2-Amino-7-carbethoxymethoxy-1,2,3,4-tetrahydronaphthalin oder eines seiner Salze ist.

3. Verbindung nach Anspruch 1, dadurch gekennzeichnet, daß sie (R)-2-Amino-7-carbethoxymethoxy-1,2,3,4-tetrahydronaphthalin oder eines seiner Salze ist.

4. Verbindung nach Anspruch 1, dadurch gekennzeichnet, daß sie (S)-2-Amino-7-carbethoxymethoxy-1,2,3,4-tetrahydronaphthalin oder eines seiner Salze ist.

5. Verfahren zur Herstellung von 2-Amino-7-hydroxytetralinethern nach Anspruch 1, dadurch gekennzeichnet, daß man ein N-geschütztes 2-Amino-7-hydroxytetralin der Formel: 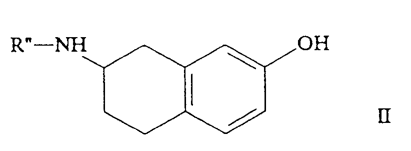 worin R'' eine N-Schutzgruppe ist, die durch katalytische Hydrierung oder durch sanfte saure Hydrolyse entfernt werden kann, mit einer Verbindung der Formel:
R'-Hal III
worin R' wie in Anspruch 1 definiert ist und Hal für Chlor, Brom oder Jod steht, in Gegenwart eines basischen Kondensiermittels behandelt, den so erhaltenen N-geschützten 2-Amino-7-hydroxytetralinether der Formel: 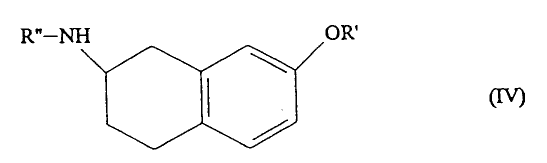 worin R' und R'' wie oben definiert sind, einer N-Entschützung durch katalytische Hydrierung oder durch sanfte saure Hydrolyse unterzieht, und, gegebenenfalls nach Verseifung der nied.Carbalcoxygruppe in eine Carboxygruppe, das Endprodukt, gegebenenfalls in Salzform, isoliert und es gegebenenfalls in eines seiner Salze überführt.

6. Verfahren nach Anspruch 5, dadurch gekennzeichnet, daß die N-Schutzgruppe ausgewählt ist aus den Gruppen tert.Butoxycarbonyl, Benzyloxycarbonyl und den Gruppen Benzyl, Benzhydryl und Trityl, die gegebenenfalls an der oder einer der Phenylgruppe(n) durch eine Methoxygruppe substituiert sind.

7. N-geschützter 2-Amino-7-hydroxytetralinether der Formel: 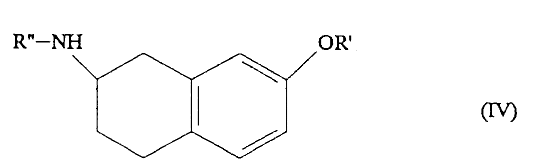 worin R' wie in Anspruch 1 definiert ist und R'' wie in Anspruch 5 definiert ist, oder eines seiner Salze.

8. Verbindung nach Anspruch 7, dadurch gekennzeichnet, daß R'' ausgewählt ist aus den Gruppen tert.Butoxycarbonyl, Benzyloxycarbonyl und den Gruppen Benzyl, Benzhydryl und Trityl, die gegebenenfalls am Phenylring durch eine Methoxygruppe substituiert sind.

9. Verbindung nach Anspruch 8, dadurch gekennzeichnet, daß sie 2-Benzylamino-7-carbethoxymethoxy-1,2,3,4-tetrahydronaphthalin oder eines seiner Salze ist.

10. Verbindung nach Anspruch 8, dadurch gekennzeichnet, daß sie 2-tert.Butoxycarbonylamino-7-carbethoxymethoxy-1,2,3,4-tetrahydronaphthalin ist.

11. Verbindung nach Anspruch 8, dadurch gekennzeichnet, daß sie 2-(R)-tert.Butoxycarbonylamino-7-carbethoxymethoxy-1,2,3,4-tetrahydronaphthalin ist.

12. Verbindung nach Anspruch 8, dadurch gekennzeichnet, daß sie 2-(S)-tert.Butoxycarbonylamino-7-carbethoxymethoxy-1,2,3,4-tetrahydronaphthalin ist.

13. Verwendung eines 2-Amino-7-hydroxytetralinethers nach einem der Ansprüche 1 bis 4 zur Herstellung des entsprechenden Phenylethanolaminotetralins der Formel: 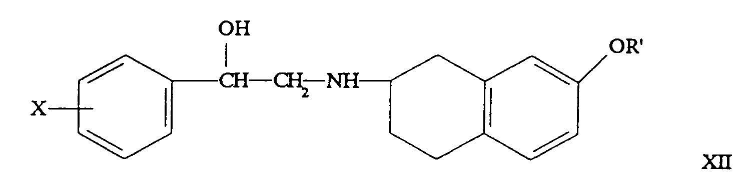 worin X Wasserstoff, ein Halogen, eine Trifluormethylgruppe oder eine nied.Alkylgruppe darstellt und R' wie in Anspruch 1 definiert ist, entweder durch Reaktion mit einem Styrolepoxid der Formel 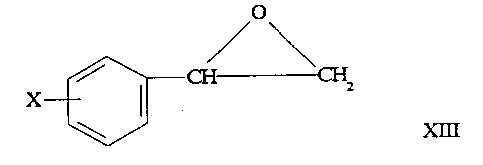 worin X wie oben definiert ist, oder durch Reaktion mit einem funktionellen Derivat einer Mandelsäure der Formel: 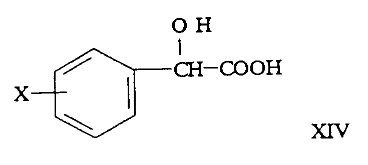 worin X wie oben definiert ist, und Reduktion der Amidcarbonylgruppe des so erhaltenen Mandelsäureamids der Formel: 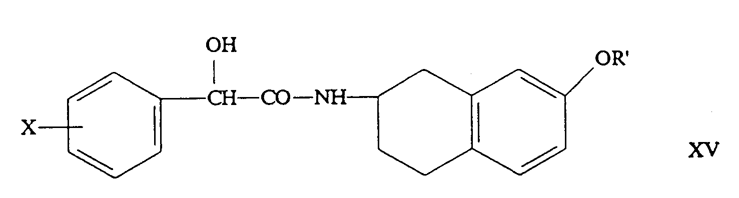 worin X und R' wie oben definiert sind, in eine Methylengruppe und gegebenenfalls Versalzung.

## Patentansprüche (Patentansprüche für folgende(n) Vertragsstaat(en): ES)

1. Verfahren zur Herstellung von 2-Amino-7-hydroxytetralinethern der Formel 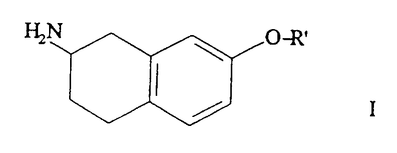 worin R' eine durch eine Carboxy- oder nied.Carbalcoxygruppe substituierte Methylgruppe darstellt, oder eines ihrer Salze, dadurch gekennzeichnet, daß man ein N-geschütztes 2-Amino-7-hydroxytetralin der Formel: 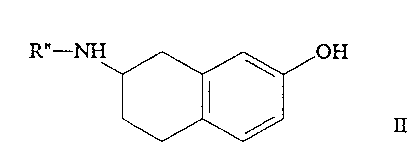 worin R'' eine N-Schutzgruppe ist, die durch katalytische Hydrierung oder durch sanfte saure Hydrolyse entfernt werden kann, mit einer Verbindung der Formel:
R'-Hal III
worin R' wie oben definiert ist und Hal für Chlor, Brom oder Jod steht, in Gegenwart eines basischen Kondensiermittels behandelt, den so erhaltenen N-geschützten 2-Amino-7-hydroxytetralinether der Formel: 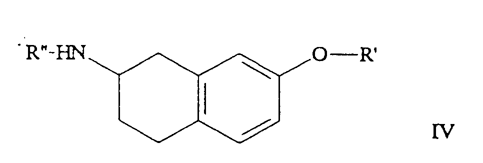 worin R' und R'' wie oben definiert sind, einer N-Entschützung durch katalytische Hydrierung oder durch sanfte saure Hydrolyse unterzieht, und, gegebenenfalls nach Verseifung der nied.Carbalcoxygruppe in eine Carboxygruppe, das Endprodukt, gegebenenfalls in Salzform, isoliert und es gegebenenfalls in eines seiner Salze überführt.

2. Verfahren nach Anspruch 1, dadurch gekennzeichnet, daß die N-Schutzgruppe ausgewählt ist aus den Gruppen tert.Butoxycarbonyl, Benzyloxycarbonyl und den Gruppen Benzyl, Benzhydryl und Trityl, die gegebenenfalls an der oder einer der Phenylgruppe(n) durch eine Methoxygruppe substituiert sind.

3. Verfahren nach Anspruch 1 zur Herstellung einer Verbindung der Formel I, worin R' eine Carbethoxymethoxygruppe ist.

4. Verfahren nach Anspruch 1 zur Herstellung einer Verbindung der Formel I, worin R' eine Carbethoxymethoxy ist und das die Aminogruppe tragende Kohlenstoffatom absolute (R)-Konfiguration hat.

5. Verfahren nach Anspruch 1 zur Herstellung einer Verbindung der Formel I, worin R' eine Carbethoxymethoxy ist und das die Aminogruppe tragende Kohlenstoffatom absolute (S)-Konfiguration hat.

6. Verfahren zur Herstellung von N-geschützten 2-Amino-7-hydroxytetralinethern der Formel IV: 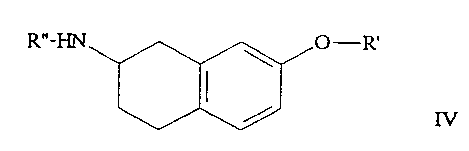 worin R' und R'' wie in Anspruch 1 definiert sind, oder eines ihrer Salze, dadurch gekennzeichnet, daß man ein N-geschütztes 2-Amino-7-hydroxytetralin der Formel II: 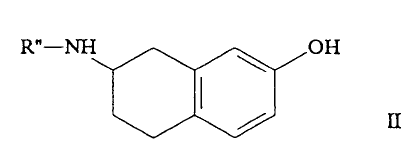 worin R'' wie oben definiert ist, mit einer Verbindung der Formel:
R'-Hal III
worin R' wie oben definiert ist und Hal für Chlor, Brom oder Jod steht, in Gegenwart eines basischen Kondensiermittels behandelt und die so erhaltene Verbindung der Formel IV, gegebenenfalls in Form eines ihrer Salze, nach herkömmlichen Methoden isoliert.

7. Verfahren nach Anspruch 6, dadurch gekennzeichnet, daß R'' ausgewählt ist aus den Gruppen tert.Butoxycarbonyl, Benzyloxycarbonyl und den Gruppen Benzyl, Benzhydryl und Trityl, die gegebenenfalls am Phenylring durch eine Methoxygruppe substituiert sind.

8. Verfahren nach Anspruch 7, dadurch gekennzeichnet, daß R' eine Carbethoxymethylgruppe und R'' ausgewählt aus den Gruppen Benzyl und tert.Butoxycarbonyl ist.

9. Verfahren nach Anspruch 7, dadurch gekennzeichnet, daß R' eine Carbethoxymethylgruppe und R'' eine tert.Butoxycarbonylgruppe ist und das die Gruppe -NHR'' tragende Kohlenstoffatom absolute (R)- oder (S)-Konfiguration hat.

10. Verwendung eines nach einem der Ansprüche 1 bis 9 hergestellten 2-Amino-7-hydroxytetralinethers zur Herstellung des entsprechenden Phenylethanolaminotetralins der Formel: 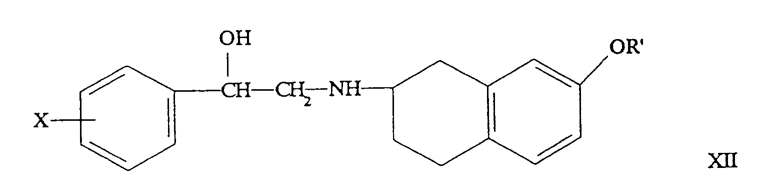 worin X Wasserstoff, ein Halogen, eine Trifluormethylgruppe oder eine nied.Alkylgruppe darstellt und R' wie in Anspruch 1 definiert ist, entweder durch Reaktion mit einem Styrolepoxid der Formel 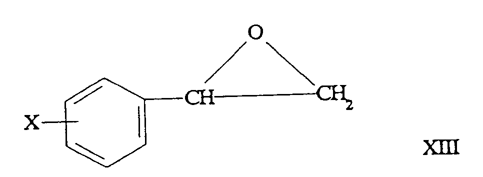 worin X wie oben definiert ist, oder durch Reaktion mit einem funktionellen Derivat einer Mandelsäure der Formel: 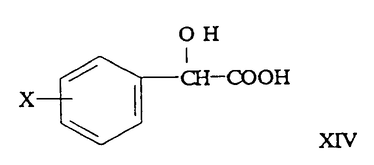 worin X wie oben definiert ist, und Reduktion der Amidcarbonylgruppe des so erhaltenen Mandelsäureamids der Formel: 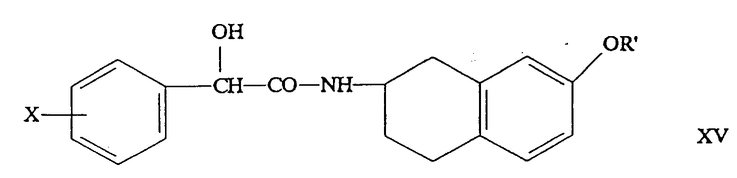 worin X und R' wie oben definiert sind, in eine Methylengruppe und gegebenenfalls Versalzung.
